# EUROPEAN PATENT APPLICATION

(11) **EP 4 656 149 A1**
(43) Date of publication of application: **03.12.2025**
(21) Application number: 24178589.8
(22) Date of filing: 28.05.2024
(51) Int. Cl.: A61B 18/14, A61M 25/01, A61B 17/3207, A61B 18/00, A61M 25/00

(54) **CATHETER ASSEMBLY FOR SLITTING A DISSECTION MEMBRANE OF AN AORTA**

(71) Applicant: Universität Linz, 4040 Linz (AT)
(72) Inventor: ZIERER, Andreas, 4040 Linz (AT)
(74) Representative: Vossius & Partner Patentanwälte Rechtsanwälte mbB

(57) **Abstract**

The invention relates to a catheter assembly for slitting a dissection membrane of an aorta of a patient which comprises an elongate first catheter (2) and an elongate second catheter (4). The elongate first catheter (2) has a lumen (24) extending therethrough and a first catheter end portion (22) configured to be inserted into a lumen of an aorta of a patient. The first catheter end portion (22) defines a first direction (d1) and comprises an opening (26). The lumen (24) of the first catheter end portion (22) opens into an external space (A) via the opening (26). The elongate second catheter (4) is disposed movably within the lumen (24) of the first catheter (2) and has a second catheter end portion (42). The second catheter end portion (42) is movable relative to the first catheter (2) between a first state and a second state. When in the first state, the second catheter end portion (42) is disposed at least essentially within the lumen (24) of the first catheter (2). When in the second state, the second catheter end portion (42) extends through the opening (26) into the external space (A) outside of the first catheter (2). The second catheter end portion (42) is flexible and has a free end (44) which defines a free end direction (df) in dependence on a flexural state of the second catheter end portion (42). The second catheter end portion (42) is configured such that, when the second catheter end portion (42) is in the first state, the free end (44) assumes a first orientation relative to the first catheter end portion (22), and, when the second catheter end portion (42) is in the second state, the free end (44) assumes a second orientation relative to the first catheter end portion (22), wherein the second orientation differs from the first orientation.

## Description

The present disclosure relates to a catheter assembly for slitting a dissection membrane of an aorta, particularly of a human aorta.

### BACKGROUND

Aortic dissection, with an incidence of 3 per 100,000 per year, is a highly acute and life-threatening clinical picture that represents the maximum manifestation of acute aortic syndrome. In this syndrome, blood accumulates via a tear in the innermost layer of the aorta between the aortic wall layers, forcing the layers apart. In the worst case, blood escapes via another tear. As a rule, however, a second parallel pathway is created. The bloodstream formed in this way is called "false lumen", the original existing bloodstream is called "true lumen". The so-called dissection membrane is located between the two lumens.

These lumens can expand aneurysmatically and rupture over time. Depending on the location, the treatment is open surgery with replacement of the aorta or the insertion of an endoprosthesis to seal the enlargement from the inside.

Often one of the two lumens is only slit-shaped. This is usually the original lumen, i. e. the true lumen, from which all arteries for organ perfusion originate. In this case, the insertion of an endoprosthesis is not possible.

US 2011/0144672 A1 relates to methods of using medical cutting tools for treating aortic septal dissections. However, experience has shown that all devices available to date lead to difficulties in practical use. For example, there is a risk that the true lumen cannot be suitably widened, which means that it is not possible to improve the blood supply to the organs or to insert a suitable endoprosthesis. There is also a risk of injuring the aortic wall with respective instruments. Furthermore, the handling of known instruments is comparatively challenging.

Therefore, there is a need for an improved catheter assembly for slitting a dissection membrane of an aorta of a patient.

These objects are achieved by the independent claims. Dependent claims refer to preferred embodiments. Additional and/or alternative aspects of the present disclosure are discussed in the specification and in the aspects.

### SUMMARY

According to the present disclosure a catheter assembly for slitting a dissection membrane of an aorta of a patient is provided which comprises an elongate first catheter and an elongate second catheter. The elongate first catheter has a lumen extending therethrough and a first catheter end portion configured to be inserted into a lumen of an aorta of a patient. The first catheter end portion defines a first direction and comprises an opening. The lumen of the first catheter end portion opens into an external space via the opening.

The elongate second catheter is disposed movably within the lumen of the first catheter and has a second catheter end portion. The second catheter end portion is movable relative to the first catheter between a first state and a second state. When in the first state, the second catheter end portion is disposed at least essentially within the lumen of the first catheter. When in the second state, the second catheter end portion extends through the opening into the external space outside of the first catheter.

The second catheter end portion is flexible and has a free end which defines a free end direction. The second catheter end portion has a central axis and the free end direction may be defined as an imaginary extension of said central axis. Said free end direction may depend on a flexural state of the second catheter end portion.

The second catheter end portion is configured such that, when the second catheter end portion is in the first state, the free end assumes a first orientation relative to the first catheter end portion, and, when the second catheter end portion is in the second state, the free end assumes a second orientation relative to the first catheter end portion, wherein the second orientation differs from the first orientation.

The flexible design of the second catheter end portion may allow the catheter assembly to have a comparatively small diameter when the second catheter end portion is in the first state, so that the catheter assembly can be advanced into an aorta, for example in a true lumen of an aorta, in a particularly suitable manner. Subsequently, when the second catheter end portion is moved from the first state to the second state, the orientation of its free end in relation to the first catheter end portion changes successively due to the flexible design of the second catheter end portion. In this way, the second catheter end portion may be particularly suitable for first puncturing the dissection membrane of the aorta and then assuming a position allowing slitting of the dissection membrane.

The second catheter end portion may be biased such that the free end assumes the second orientation relative to the first catheter end portion when the second catheter end portion is in the second state; particularly with no external forces acting on the second catheter end portion. In this way, it may be possible to achieve that the free end changes its orientation relative to the first catheter end portion autonomously during or upon movement of the second catheter end portion from the first state to the second state. This may contribute in particular to easier handling during operation. The bias may be achieved, for example, by manufacturing the second catheter end portion from a suitable material.

The configuration may be such that when the second catheter end portion is in the second state, the free end direction is at least essentially opposite to the first direction. This may allow the first catheter or its end portion and the second catheter end portion to define or form a kind of a U-shape or "hook". This may be advantageous because in this way the two U-shaped legs may be used extend on opposite sides of the dissection membrane and opposite to one another to facilitate slitting from two opposite sides, i. e. from both the false lumen and from the true lumen, allowing the slitting process to be carried out in a particularly guided and targeted manner. The slitting process may be carried out by pulling the catheter assembly in a direction in which the free ends of the two U-legs point. In other words, the slitting direction may be towards the open-side of the U-shaped configuration. In this way, the slitting process may be caried out by pulling the catheter assembly in a direction that corresponds to the main direction of blood flow in the aorta.

The configuration may be such that when the second catheter end portion is in the second state, the angle defined between the first direction and the free end direction may be between 90° and 180°, preferably between 120° and 180°. The angle is measured as the orientational deviation of the second catheter end portion from the first catheter end portion while assuming the angle to be zero when the second catheter end portion and the first catheter end portion are co-aligned and point in the same direction; in other words, when the first direction and the free end direction are identical. An angle of 180° thus means that the first direction and the free end direction point in opposite directions. This means that at least a V-shape may be formed with the two respective parts, i. e. the first catheter or its end portion on the one side and the second catheter end portion on the other side. This may be advantageous with respect to the achievable precision of the slitting process.

The configuration may be such that when the second catheter end portion is in the first state, the free end direction is at least essentially co-aligned with the first catheter end portion (in this state, the angle may be 0°). In this way, it may be achieved that the catheter assembly assumes a substantially cylindrical shape, in particular a circular cylindrical shape, extending along the first direction when the second catheter end portion is in its first state, making it particularly easy to insert the catheter assembly into the aorta and to advance the assembly through the aorta.

The first catheter end portion may comprise a tubular side wall, wherein the opening is an opening formed in the tubular side wall. In other words, the opening may be a side-facing opening with respect to the first direction. There may be no opening in the catheter top, i.e., in the first direction and/or there may only be one opening. By means of the provision of an opening in the tubular side wall, it may be achieved that the first catheter end portion has a region protruding beyond the opening in the first direction, which may be used to attach positioning support elements such as for example wing elements thereto, with which a desired positioning of the first catheter end portion within the aorta can be facilitated and/or supported.

The first catheter end portion may comprise a guiding structure configured to laterally deflect the free end of the second catheter end portion with respect to the first direction, when the second catheter end portion is moved from the first state into the second state. In this way, the second catheter end portion may be moved in a particularly guided manner in relation to the first catheter end portion. This also may facilitate handling of the catheter assembly during operation.

The first catheter may be configured to guide a contrast medium or contrast agent through the lumen and out of the opening to the external space. By injecting a contrast medium out of the opening of the first catheter end portion, the aorta or the corresponding aortic area - for example an area within the true lumen - may be made more visible in the X-ray image during a respective procedure.

The catheter assembly may further comprise a cutting wire which extends through the lumen of the first catheter. The cutting wire may not extend through the lumen of the second catheter. The cutting wire may be fixed to a fixation point on the second catheter end portion. In this way, when the second catheter end portion is in the second state, the cutting wire may extend at least essentially straight between the first catheter end portion and the second catheter end portion. The cutting wire may be configured to be energized with electrical current.

The cutting wire may be configured to slit the dissection membrane of the aorta via electrical current. In this way, the cutting wire may be used to slit the dissection membrane via electrocautery.

The cutting wire may be configured to stabilize the free end of the second catheter end portion in the second orientation relative to the first catheter or its end portion. In this way, the alignment of the free end in relation to the first catheter end portion may be additionally mechanically stabilized. This may be particularly advantageous with respect to the achievable accuracy of the slitting process.

The cutting wire may have a diameter of between 0.15 mm and 0.5 mm, preferably between 0.2 mm and 0.4 mm. These diameter ranges have proven to be particularly suitable for slitting a dissection membrane.

The cutting wire may extend partially through the lumen of the first catheter, and preferably outside the lumen of the second catheter. The cutting wire may be movably disposed within said first catheter's lumen. This may be advantageous for manipulating the cutting wire, for example via a handling element such as for example a manipulation handle which is disposed at an end of the catheter assembly opposite to the first catheter end portion, i. e., at an end of the catheter assembly that is configured to be positioned outside the patient when operating the catheter assembly.

The configuration may be such that when the free end is in the second orientation, the cutting wire extends through the opening of the first catheter end portion into the external space and to the fixation point on the second catheter end portion. The configuration is preferably such that a portion of the cutting wire that extends from the opening to the fixation point defines a cutting wire direction from the opening to the fixation point that takes an angle (in line with the above discussion) with the first direction that is greater than 90°. In other words, the guiding wire takes a slanted, non-normal orientation to the dissection membrane and/or the cutting direction. In this way, the dissection membrane may be guided against the first catheter end portion during the slitting process. In particular, this may allow the dissection membrane to be guided to the point at which the cutting wire exits the opening of the first catheter end portion during the slitting process in the sense of self-centering. This may enable the slitting process to be carried out with particular precision.

The cutting wire may comprise a cutting area which is configured for cutting the dissection membrane. For example, the above-mentioned portion of the cutting wire extending from the opening to the fixation point may constitute the cutting area of the cutting wire.

The cutting wire outside the cutting area may be provided with an electrically insulating cutting wire coating. In this way, the risk of unintentional damage to aortic tissue may be avoided or at least reduced.

Preferably, the cutting wire coating is flexible and temperature-stable.

The opening of the first catheter end portion may comprises a notch for guiding the cutting wire. In this way, the wire may be positioned particularly precisely and/or stably at the location of the opening.

The free end of the second catheter end portion may comprise a puncture tip configured to puncture the dissection membrane of the aorta.

In particular, the configuration may be such that as the second catheter end portion moves from its first state to its second state, the free end moves relative to the first catheter end portion such that the angle between the first direction and the free end direction increases. In other words, the free end increasingly deviates from the first direction during the transition of the second catheter end portion from the first state to the second state.

Accordingly, it may be achieved that the free end in a certain intermediate position of the second catheter end portion between its first and second state is oriented particularly suitably for puncturing the dissection membrane.

The puncture tip may be configured to be energized with electrical current such that the dissection membrane can be punctured by the puncture tip via electrical current.

The catheter assembly may further comprise a puncture tip wire. The puncture tip wire may be configured for supplying electrical energy to the puncture tip.

The puncture tip wire may have a diameter of between 0.15 mm and 0.5 mm, preferably between 0.2 mm and 0.4 mm.

The second catheter may have a lumen that extends therethrough, wherein the puncture tip wire is disposed movably within the lumen of the second catheter. For example, the puncture tip wire may extend to the manipulation handle. This may be advantageous for manipulating the puncturing tip or for applying current to the puncturing tip, for example via the manipulation handle which may be disposed at an end of the catheter assembly opposite to the first catheter end portion.

The second catheter may be configured to guide a contrast medium through the lumen and out of an opening of the second catheter end portion to the external space. By injecting a contrast medium out of the opening of the second catheter end portion, the aorta or the corresponding aortic area - for example an area of the false lumen - may be made more visible in the X-ray image during a respective procedure.

The puncture tip wire may comprise an electrically insulating puncture tip wire coating which is flexible, and temperature-stable. In this way, the risk of unintentional damage to aortic tissue may be avoided or at least reduced.

The second catheter may comprise a tube-shaped outer wall element which is made at least partly from an electrical insulating material. The wall element may be made of plastic, for example. This also may be advantageous for achieving the afore-mentioned bias of the second catheter end portion.

The second catheter may comprise a spacer element that is configured to be moved radially inwards and outwards with respect the free end direction between a first, radially retracted position and a second, radially extended position.

The configuration may be such that when in the radially extended position, the second catheter spacer element is configured to hold the second catheter end portion and the cutting wire in a desired position relative to the dissection membrane.

The second catheter spacer spacer element may comprise two wing elements which are arranged on two opposite sides with respect to the free end direction.

The two wing elements may be arranged normal to a plane defined by the first direction and a section or portion of the cutting wire that extends between the second catheter end portion and the first catheter.

The wing elements may be formed by longitudinal slots within the wall of the second catheter end portion, preferably by slots extending in the free end direction.

The configuration may be such that the second catheter spacer element can be moved from its first, radially retracted position into its second, radially extended position by manipulating the puncture tip wire. The manipulation may for example comprise pulling the puncture tip wire, for example using the manipulation handle.

The catheter assembly may further comprise an elongate third catheter that has a lumen extending therethrough. The first catheter is preferably disposed at least partially within the lumen of the third catheter. The third catheter may comprise a spacer element that is configured to be moved radially inwards and outwards with respect to the first direction between a first, radially retracted position and a second, radially extended position, wherein, when in the radially extended position the spacer element is configured to hold the first catheter end portion in a desired position relative to the lumen of the aorta.

The third catheter spacer element may comprise at least two wing elements formed by longitudinal slots within the wall of the third catheter, preferably by slots extending in the first direction.

The third catheter may be fixed to the first catheter end portion in an area between the longitudinal slots and a free end of the third catheter.

The third catheter may comprise a movable section located on the opposite side of the longitudinal slots to the area where the third catheter is fixed to the first catheter in relation to the longitudinal slots. The movable section is movable relative to the first catheter, wherein the spacer element can be moved from its first, radially retracted position into its second, radially extended position by moving the movable section of the third catheter relative to the first catheter. For example the movable section may be moved towards the area where the third catheter is fixed to the first catheter end portion.

The third catheter may comprise an opening which is co-aligned with the opening of the first catheter end portion. The opening may be elongate such that the opening is aligned with the opening of the first catheter end portion both when the spacer element is in its first, radially retracted position and when it is in its second, radially extended position.

The third catheter may be configured such that the second catheter end portion can be moved from its first state to its second state by moving through the opening of the third catheter. Preferably, the third catheter is configured such that the second catheter end portion can be moved from its first state to its second state by moving through the opening of the first catheter end portion and through the opening of the third catheter.

The third catheter may have an outer diameter of between 3 mm and 5 mm, preferably between 3 mm and 4.5 mm.

The catheter assembly may further comprise a manipulation handle that is coupled to the first catheter and configured to be disposed extracorporeal with respect to the patient.

The manipulation handle may comprise means for moving the second catheter relative to the first catheter.

The manipulation handle may comprise means for locking a position of the second catheter relative to the first catheter.

The manipulation handle may comprise means for moving the cutting wire relative to the first catheter.

The manipulation handle may comprise means for locking a position of the cutting wire relative to the first catheter.

The manipulation handle may comprise means for moving the puncture tip wire relative to the second catheter.

The manipulation handle may comprise means for locking a position of the puncture tip wire relative to the second catheter.

The manipulation handle may comprise means for moving the third catheter relative to the first catheter.

The manipulation handle may comprise means for locking a position of the third catheter relative to the first catheter.

In particular, the present disclosure comprises the following aspects:

### ASPECTS

1. A catheter assembly for slitting a dissection membrane of an aorta of a patient, comprising
   an elongate first catheter (2), having a lumen (24) extending therethrough and a first catheter end portion (22) configured to be inserted into a lumen of an aorta of a patient, the first catheter end portion (22) defining a first direction (d1) and comprising an opening (26), wherein the lumen (24) opens into an external space (A) via the opening (26),
   an elongate second catheter (4) disposed movably within the lumen (24) of the first catheter (2) and having a second catheter end portion (42), the second catheter end portion (42) being movable relative to the first catheter (2) between a first state and a second state, wherein, when in the first state, the second catheter end portion (42) is disposed at least essentially within the lumen (24) of the first catheter (2), and, when in the second state, the second catheter end portion (42) extends through the opening (26) into the external space (A) outside of the first catheter (2),
   wherein the second catheter end portion (42) is flexible and has a free end (44) defining a free end direction (df), preferably in dependence on a flexural state of the second catheter end portion (42), and
   wherein the second catheter end portion (42) is configured such that, when the second catheter end portion (42) is in the first state, the free end (44) assumes a first orientation relative to the first catheter end portion (22), and, when the second catheter end portion (42) is in the second state, the free end (44) assumes a second orientation relative to the first catheter end portion (22), wherein the second orientation differs from the first orientation.
2. The catheter assembly of aspect 1,
   wherein the second catheter end portion (42) is biased such that the free end (44) assumes the second orientation relative to the first catheter end portion (22) when the second catheter end portion (42) is in the second state, preferably with no external forces acting on the second catheter end portion (42).
3. The catheter assembly of any of the preceding aspects,
   wherein, when the second catheter end portion (42) is in the second state, the free end direction (d1) is at least essentially opposite to the first direction (d1).
4. The catheter assembly of any of the preceding aspects,
   wherein, when the second catheter end portion (42) is in the second state, the angle (α) defined between the first direction (d1) and the free end direction (df) is between 90° and 180°, preferably between 120° and 180°.
5. The catheter assembly of any of the preceding aspects,
   wherein, when the second catheter end portion (42) is in the first state, the free end direction (df) is at least essentially co-aligned with the first catheter end portion (22) and/or with the first direction (d1).
6. The catheter assembly of any of the preceding aspects,
   wherein the first catheter end portion (22) comprises a tubular wall, and wherein the opening (26) is an opening formed in the tubular wall.
7. The catheter assembly of any of the preceding aspects,
   wherein the first catheter end portion (22) comprises a guiding structure (28) configured to laterally deflect the free end (44) of the second catheter end portion (42) with respect to the first direction (d1), when the second catheter end portion (42) is moved from the first state into the second state.
8. The catheter assembly of any of the preceding aspects,
   wherein the first catheter (2) is configured to guide a contrast medium through the lumen (24) and out of the opening (26) to the external space (A).
9. The catheter assembly of any of the preceding aspects, further comprising
   a cutting wire (6) extending through the lumen (24) of the first catheter (2), the cutting wire (6) being fixed to a fixation point (64) on the second catheter end portion (42) and being configured to be energized with electrical current.
10. The catheter assembly of aspect 9,
   wherein the cutting wire (6) is configured to slit a dissection membrane of the aorta via electrical current.
11. The catheter assembly of any of aspects 9 to 10,
   wherein the cutting wire (6) is configured to stabilize the free end (44) in the second orientation relative to the first catheter (2).
12. The catheter assembly of any of aspects 9 to 11,
   wherein the cutting wire (6) has a diameter of between 0.15 mm and 0.5 mm, preferably between 0.2 mm and 0.4 mm.
13. The catheter assembly of any of aspects 9 to 12,
   wherein the cutting wire (6) extends partially through the lumen (24) of the first catheter (2) and is movably disposed within said lumen (24).
14. The catheter assembly of any of aspects 9 to 13,
   wherein, when the free end (44) is in the second orientation, the cutting wire (6) extends through the opening (26) of the first catheter end portion (22) into the external space (A) and to the fixation point (64) on the second catheter end portion (42).
15. The catheter assembly of any of aspects 9 to 14,
   wherein the cutting wire (6) comprises a cutting area which is configured for cutting the dissection membrane.
16. The catheter assembly of any of aspects 9 to 15,
   wherein the cutting wire (6) outside the cutting area is provided with an electrically insulating cutting wire coating.
17. The catheter assembly of aspect 16,
   wherein the cutting wire coating is flexible and temperature-stable.
18. The catheter assembly of any of aspects 9 to 17,
   wherein the opening (26) comprises a notch (27) for guiding and/or positioning the cutting wire (6).
19. The catheter assembly of any of aspects 1 to 18,
   wherein the free end (44) of the second catheter end portion (42) comprises a puncture tip (46) configured to puncture the dissection membrane of the aorta.
20. The catheter assembly of aspect 19,
   wherein the puncture tip (46) is configured to be energized with electrical current such that the dissection membrane can be punctured by the puncture tip (46) via electrical current.
21. The catheter assembly of any of aspects 19 to 20, further comprising
   a puncture tip wire (48) for supplying electrical energy to the puncture tip (46).
22. The catheter assembly of aspect 21,
   wherein the puncture tip wire (48) has a diameter of between 0.15 mm and 0.5 mm, preferably between 0.2 mm and 0.4 mm.
23. The catheter assembly of any of aspects 21 to 22,
   wherein the second catheter (4) has a lumen (41) extending therethrough, wherein the puncture tip wire (48) is disposed movably within the lumen (41) of the second catheter (4).
24. The catheter assembly of aspect 23,
   wherein the second catheter (4) is configured to guide a contrast medium through the lumen (41) and out of an opening (45) of the second catheter end portion (42) to the external space (A).
25. The catheter assembly of any of aspects 21 to 24,
   wherein the puncture tip wire (48) comprises an electrically insulating puncture tip wire coating which is flexible, and temperature-stable.
26. The catheter assembly of any of aspects 1 to 25,
   wherein the second catheter (4) comprises a tube-shaped outer wall element which is made at least partly from an electrical insulating material.
27. The catheter assembly of any of aspects 1 to 26,
   wherein the second catheter (2) comprises a spacer element (27) configured to be moved radially inwards and outwards with respect the free end direction (df) between a first, radially retracted position and a second, radially extended position.
28. The catheter assembly of aspect 27,
   wherein, when in the radially extended position, the spacer element (27) is configured to hold the second catheter end portion (42) and the cutting wire (6) in a desired position relative to the dissection membrane.
29. The catheter assembly of any of aspects 27 to 28,
   wherein the spacer element (27) comprises two wing elements (272, 274) which are arranged on two opposite sides with respect to the free end direction (df).
30. The catheter of aspect 29,
   wherein the two wing elements (272, 274) are arranged normal to a plane defined by the first direction (d1) and a section of the cutting wire (6) that is between the second catheter end portion (42) and the first catheter (2).
31. The catheter assembly of any of aspects 29 to 30,
   wherein the wing elements (272, 274) are formed by longitudinal slots within the second catheter end portion (42), preferably by slots extending in the free end direction (df).
32. The catheter assembly of any of aspects 27 to 31, comprising the features of aspect 21,
   wherein the spacer element (27) can be moved from its first, radially retracted position into its second, radially extended position by manipulating the puncture tip wire (48).
33. The catheter assembly of any of aspects 1 to 32, further comprising
   an elongate third catheter (5), having a lumen extending therethrough, wherein the first catheter (2) is preferably disposed at least partially within the lumen of the third catheter (5),
   the third catheter (5) comprising a spacer element (57) configured to be moved radially inwards and outwards with respect to the first direction (d1) between a first, radially retracted position and a second, radially extended position, wherein, preferably, when in the radially extended position the spacer element (57) is configured to hold the first catheter end portion (22) in a desired position relative to the lumen of the aorta.
34. The catheter assembly of aspect 33,
   wherein the spacer element (57) comprises at least two wing elements (572, 574, 576, 578) formed by longitudinal slots within the third catheter (5), preferably by slots extending in the first direction (d1).
35. The catheter assembly of aspect 34,
   wherein the third catheter (5) is fixed to the first catheter end portion (22) in an area (52) between the longitudinal slots and a free end (53) of the third catheter (5).
36. The catheter assembly of aspect 35,
   wherein the third catheter (5) comprises a movable section opposite to the area (52) with respect to the slots which is movable relative to the second catheter (2), wherein the spacer element (57) can be moved from its first, radially retracted position into its second, radially extended position by moving the movable section of the third catheter (5) relative to the second catheter (2).
37. The catheter assembly of any of aspects 33 to 36,
   wherein the third catheter (5) comprises an opening (54) which is co-aligned with the opening (26) of the first catheter end portion (22).
38. The catheter assembly of aspect 37,
   wherein the third catheter (5) is configured such that the second catheter end portion (42) can be moved from its first state to its second state by moving through the opening (54) of the third catheter (5).
39. The catheter assembly of any of aspects 33 to 38,
   wherein the third catheter (5) has an outer diameter of between 3 mm and 5 mm, preferably between 3 mm and 4.5 mm.
40. The catheter assembly of any of aspects 1 to 39, further comprising
   a manipulation handle (3), coupled to the first catheter (2) and configured to be disposed extracorporeal with respect to the patient.
41. The catheter assembly of aspect 40,
   wherein the manipulation handle (3) comprises means for moving the second catheter (4) relative to the first catheter (2).
42. The catheter assembly of aspect 41,
   wherein the manipulation handle (3) comprises means for locking a position of the second catheter (4) relative to the first catheter (2).
43. The catheter assembly of any of aspects 40 to 42,
   wherein the manipulation handle (3) comprises means for moving the cutting wire (6) relative to the first catheter (2).
44. The catheter assembly of aspect 43,
   wherein the manipulation handle (3) comprises means for locking a position of the cutting wire (6) relative to the first catheter (2).
45. The catheter assembly of any of aspects 40 to 44, comprising the features of aspect 21,
   wherein the manipulation handle (3) comprises means for moving the puncture tip wire (48) relative to the second catheter (4).
46. The catheter assembly of aspect 45,
   wherein the manipulation handle (3) comprises means for locking a position of the puncture tip wire (48) relative to the second catheter (4).
47. The catheter assembly of any of aspects 40 to 46, comprising the features of aspect 31, wherein the manipulation handle (3) comprises means for moving the third catheter (5) relative to the first catheter (2).
48. The catheter assembly of aspect 47,
   wherein the manipulation handle (3) comprises means for locking a position of the third catheter (5) relative to the first catheter (2).

### SHORT DESCRIPTION OF THE DRAWINGS

The subject-matter of the disclosure will be explained in more detail with reference to preferred exemplary embodiments which are illustrated in the attached drawings, in which:
Fig. 1 is a schematic perspective view of an end portion of a catheter assembly according to the present description in a state configured for advancing through an aorta, showing a first or middle catheter with a lumen extending therethrough, a second or inner catheter disposed movably in the lumen of the first catheter, and a third or outer catheter surrounding the first catheter.
Fig. 2 shows a corresponding cross section.
Fig. 3 shows the end portion of the catheter assembly in a state with extended spacer or wing elements formed by the third or outer catheter.
Fig. 4 shows a corresponding cross section.
Fig. 5 shows the end portion of the catheter assembly in a state with an end portion of the second or inner catheter extending through an opening of the first or middle catheter.
Fig. 6 shows a corresponding view, however, with extended spacer elements formed by the second or inner catheter.
Fig. 7 shows a corresponding cross section.
Fig. 8 shows the view comparable to that of Fig. 6 somewhat more detailed.
Fig. 9 shows a detail of Fig. 8.
Fig. 10 is a schematic illustration of the catheter assembly, including a manipulation handle.
Fig. 11 shows a perspective view of a catheter assembly according to a further example, in a state with an end portion of the second or inner catheter extending through an opening of the first or middle catheter and with extended spacer or wing elements formed by the third or outer catheter.
Fig. 12 shows the catheter assembly of Fig. 11 with the end portion of the second or inner catheter being in a state where a free end thereof points essentially in a direction opposite to the direction defined by the middle or first catheter.
Fig. 13 shows the catheter assembly of Fig. 11 in a state configured for advancing through an aorta.
Fig. 14 shows the end portion of the catheter assembly in a state with extended spacer or wing elements formed by the third or outer catheter.

### DETAILED DESCRIPTION

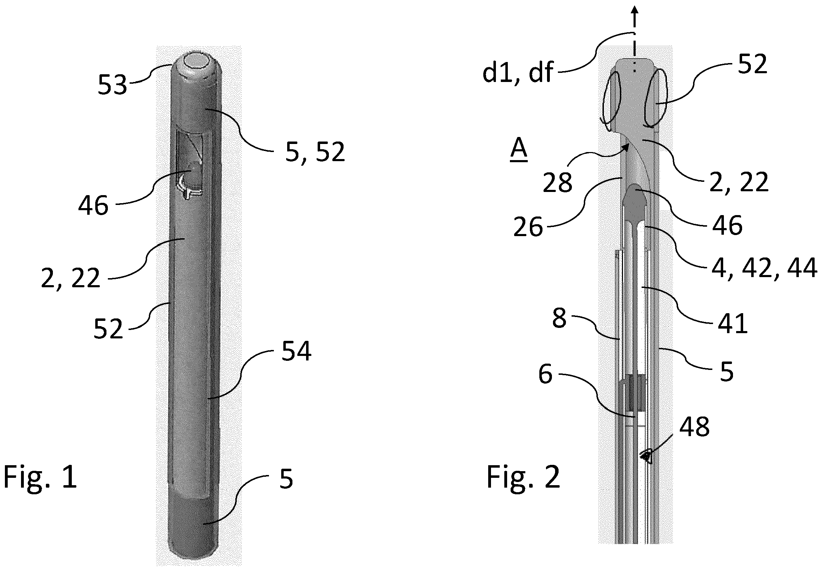

Fig. 1 is a schematic perspective view of an end portion of a catheter assembly according to the present description. The catheter assembly is configured for slitting a dissection membrane of an aorta of a patient. Fig. 2 shows a corresponding cross section.

The catheter assembly comprises an elongate first catheter 2, also referred to herein as "middle catheter" or "sheath catheter". The first catheter 2 has a lumen 24 extending therethrough. The first catheter 2 comprises a first catheter end portion 22 that is configured to be inserted into a lumen of an aorta of a patient.

More generally, the catheter assembly is configured to be inserted into an aorta, particularly into a true lumen of an aorta descendens, for example via a femoral access sheath.

The first catheter end portion 22 defines a first direction d1 also referred to herein as advancing direction because the catheter assembly is configured to be advanced for insertion into the aorta in this first direction d1 inside the patient's body. The direction d1 may be in line with the extension of an end portion's 22 central axis (not shown).

The first catheter end portion 22 comprises an opening 26, wherein the lumen 24 of the first catheter 2 opens into an external space A via the opening 26. "External space" here refers to a space outside the catheter assembly. Opening 26 is provided in the catheter's side wall, the opening extending parallel to the catheter's longitudinal axis. In other words, it does not cross said longitudinal axis.

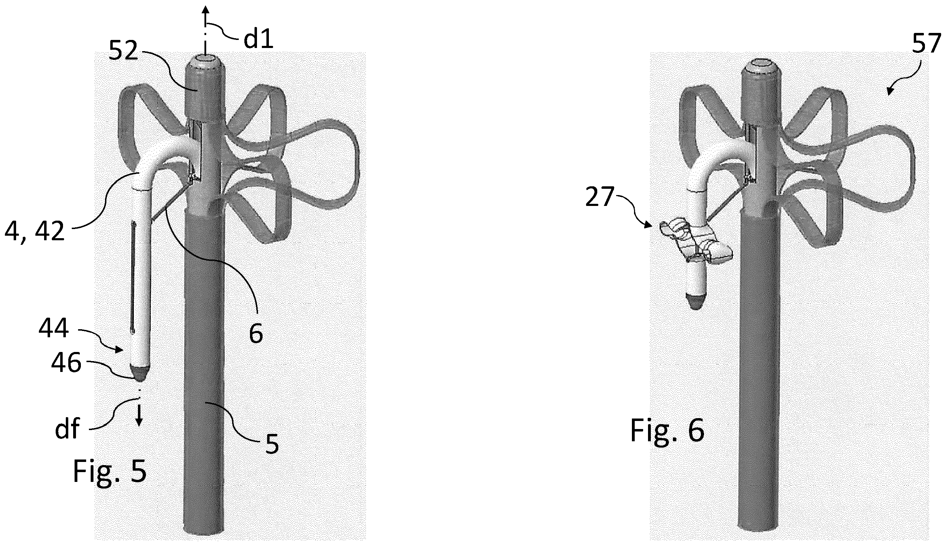

The catheter assembly further comprises an elongate second catheter4, also referred to herein as "inner catheter" or "working catheter". The second catheter 4 is disposed movably within the lumen 24 of the first catheter 2. The second catheter 4 has a second catheter end portion 42 that is movable relative to the first catheter 2 between a first state and a second state. When in the first state, the second catheter end portion 42 is disposed at least essentially within the lumen 24 of the first catheter 2. When in the second state, the second catheter end portion 42 extends through the opening 26 into the external space A outside of the first catheter 2. Figures 1 and 2 show the second catheter end portion 42 in the first state, Fig. 5, for example, shows the second catheter end portion 42 in the second state.

The second catheter end portion 42 is flexible and has a free end 44 defining a free end direction df, referenced for example in Fig. 5. Said direction may change, in dependence on a flexural state of the second catheter end portion 42.

The second catheter end portion 42 is configured such that, when the second catheter end portion 42 is in the first state, the free end 44 assumes a first orientation relative to the first catheter end portion 22, and, when the second catheter end portion 42 is in the second state, the free end 44 assumes a second orientation relative to the first catheter end portion 22, wherein the second orientation differs from the first orientation. For example, as sketched in Fig. 2, when the second catheter end portion 42 is in the first state, the free end 44 may assume a first orientation relative to the first catheter end portion 22 in which the free end 44 points in the first direction d1. Accordingly, the free end direction df may be at least essentially co-aligned with the first catheter end portion 22, i. e. with the first direction d1. When the second catheter end portion 42 is in the second state, as sketched in Fig. 5, the free end 44 may assume a second orientation relative to the first catheter end portion 22 in which the free end 44 points into a direction df at least essentially opposite to the first direction d1.

Preferably, the second catheter end portion 42 is biased such that the free end 44 assumes the second orientation relative to the first catheter end portion 22 when the second catheter end portion 42 is in the second state with no external forces acting on the second catheter end portion 42.

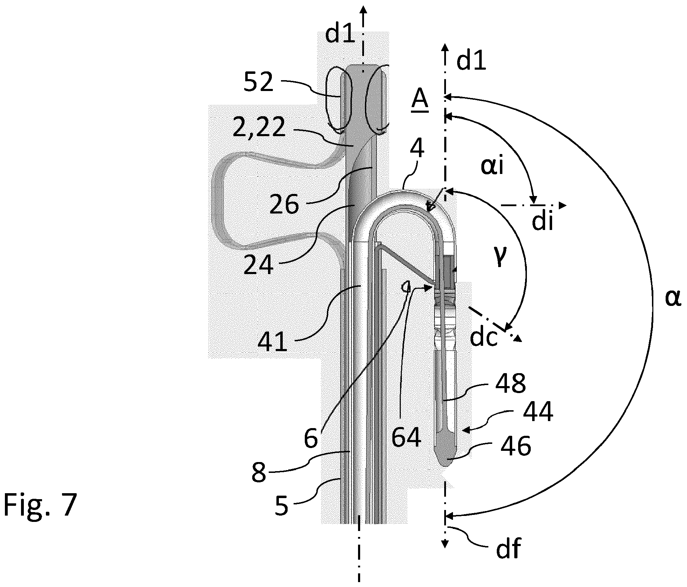

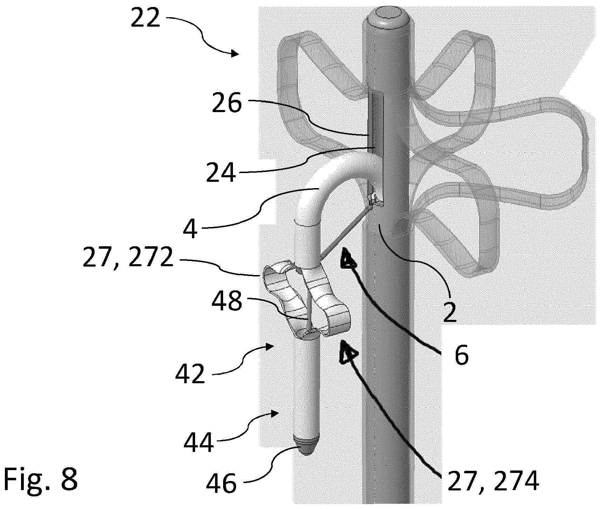

Fig. 7 shows a cross-sectional view of the end portion of the catheter assembly. Fig. 8 shows a corresponding perspective view. Preferably, the configuration is such that as the second catheter end portion 42 moves from its first state to its second state, its free end 44 moves relative to the first catheter end portion 22 - and, correspondingly, its direction may change relative to the first direction d1- such that an angle α between the first direction d1 and the free end direction df increases, preferably continuously, starting from 0° (when first direction d1 and free end direction df are algined). In other words, the free end 44 increasingly deviates from the first direction d1 during the transition of the second catheter end portion 42 from the first state to the second state.

Correspondingly, there may be an intermediate position where the angle is 90°, as referenced in Fig. 7 by α1, i. e., where the free end 44 points in a direction di that is perpendicular to the first direction di and radially outwardly from the first catheter's end portion 22.

Fig. 7 shows an example in which the free end 44 has assumed an orientation vis-à-vis the first catheter end portion 22 in which it points opposite to the first direction d1. However, more generally, it is preferred, that the angle α defined between the first direction d1 and the free end direction df is between 90° and 180°, preferably between 120° and 180°, when the second catheter end portion 42 is in the second state.

As illustrated for example in Fig. 2, the first catheter end portion 22 may comprise a tubular wall, wherein the opening 26 is an opening formed in the tubular wall. In other words, the opening 26 is a side-facing opening with reference to the first direction d1.

Further, the first catheter end portion 22 comprises a guiding structure 28 configured to laterally deflect or to assist in laterally deflecting the free end 44 of the second catheter end portion 42 with respect to the first direction d1, when the second catheter end portion 42 is moved from the first state into the second state. The guiding structure 28 may be formed for example by a guiding surface that is slanted relative to the first direction d1 or conical in shape.

The catheter assembly further comprises a cutting wire 6 that extends through the lumen 24 of the first catheter 2. The cutting wire 6 may have a diameter of between 0.2 mm and 0.4 mm.

The cutting wire 6 is fixed to a fixation point 64 on the second catheter end portion 42. The cutting wire is configured to be energized with electrical current and configured to slit the dissection membrane of the aorta via the electrical current. Correspondingly, the cutting wire 6 extends through the opening 26 of the first catheter end portion 22.

The cutting wire 6 extends partially through the lumen 24 of the first catheter 2 and is movably disposed within said lumen 24. When the free end 44 is in the second orientation, the cutting wire 6 extends through the opening 26 of the first catheter 2 into the external space A and to the fixation point 64 on the second catheter end portion 42. Accordingly, as can be taken from Fig. 7, a portion of the cutting wire 6 that extends from the opening 26 of the first catheter end portion 22 to the fixation point 64 at the second catheter end portion 42 defines a cutting wire direction dc pointing from the opening 26 to the fixation point 64 that makes an angle γ with the first direction d1 that is greater than 90°. In this way, the dissection membrane may be guided against the first catheter end portion 22 during the slitting process.

The cutting wire 6 comprises a cutting area which is configured for cutting or slitting the dissection membrane. For example, the above-mentioned portion of the cutting wire 6 that extends from the opening 26 to the fixation point 64 may form the cutting area. Preferably, the cutting wire 6 outside the cutting area is provided with an electrically insulating cutting wire coating.

Further, the cutting wire 6 may be configured to stabilize the free end 44 in its second orientation relative to the first catheter 2 when the second catheter end portion 42 is in the second state.

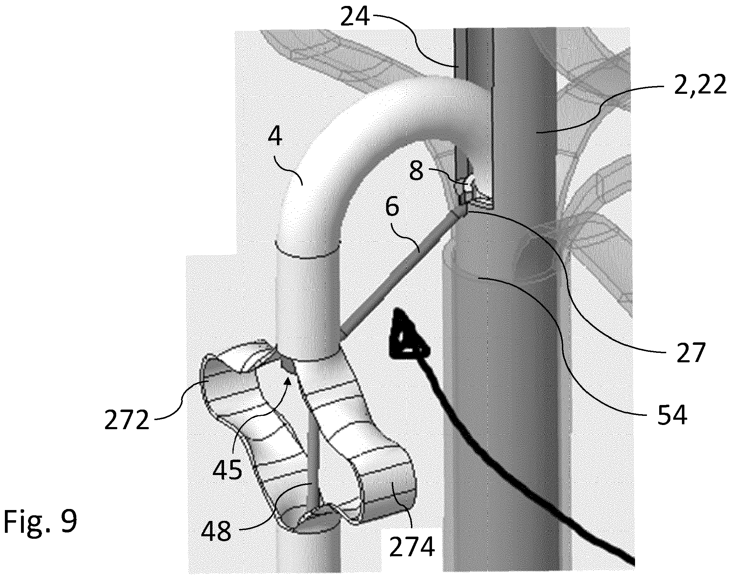

The opening 26 may comprise a notch 27 for improved guidance the cutting wire 6. The notch 27 is also illustrated in Fig. 9 that shows a detail of Fig. 8. For even more suitable guidance of the cutting wire 6 within the first catheter 2, the catheter assembly may further comprise a guide catheter 8 disposed radially between the first catheter 2 and the second catheter 4. The guide catheter 8 may comprise a longitudinal groove for guiding the cutting wire 6 therein.

The free end 44 of the second catheter end portion 42 comprises a puncture tip 46 configured to puncture the dissection membrane of the aorta. For example, the puncture tip 46 may be used to puncture the dissection membrane when the second catheter end portion 42 is in the above-mentioned intermediate position, where the free end 44 points in a direction di perpendicular to the first direction d1.

The puncture tip 46 is configured to be energized with electrical current such that the dissection membrane can be punctured by the puncture tip 46 via the electrical current. Electrical energy may be supplied to the puncture tip 48 via a puncture tip wire 48 which may be disposed movably within a lumen 41 of the second catheter 4. Preferably, the second catheter 4 comprises a tube-shaped outer wall element which is made at least partly from an electrical insulating material.

The puncture tip wire 48 preferably comprises an electrically insulating puncture tip wire coating which is flexible, and temperature-stable. The puncture tip wire 48 may have a diameter of between 0.2 mm and 0.4 mm.

Further, the second catheter 4 comprises a spacer element 27 configured to be moved radially inwards and outwards with respect the free end direction df between a first, radially retracted position - as illustrated exemplarily in Fig. 5 - and a second, radially extended position - as illustrated exemplarily in Fig. 6. When in the radially extended position, the anchor element 27 is configured to hold the second catheter end portion 42 and the cutting wire 6 in a desired position relative to the dissection membrane. The spacer element 27 comprises two wing elements 272, 274 which are arranged on two opposite sides with respect to the free end direction df. The wing elements 272, 274 may be formed by slots formed in the catheter's tubular wall extending along the free end direction df. The spacer element 6 may be moved from its radially retracted position to its radially extended position by pulling the puncture tip wire 48.

The anchor element 27 is preferably disposed or formed between the fixation point 64 of the cutting wire 6 and the puncture tip 46.

The catheter assembly further comprises an elongate third catheter 5, also referred to herein as "outer catheter" or "spacer catheter". The third catheter 5 has a lumen extending therethrough, wherein the first catheter 2 is disposed at least partially within the lumen of the third catheter 5.

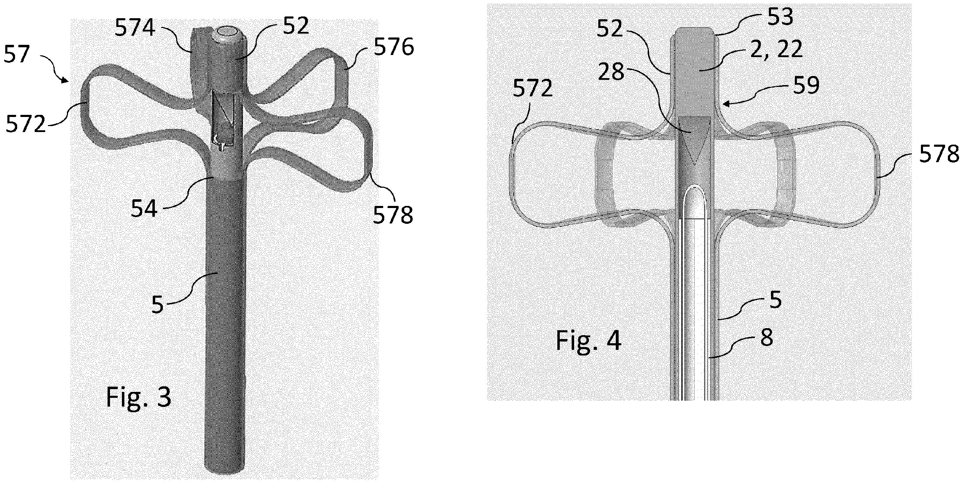

The third catheter 5 comprises a spacer element 57 configured to be moved radially inwards and outwards with respect to the first direction d1 between a first, radially retracted position - as illustrated exemplarily in Fig. 1 - and a second, radially extended position - as illustrated exemplarily in Fig. 3. When in the radially extended position, the spacer element 57 is configured to hold the first catheter end portion 22 in a desired position relative to the lumen of the aorta. Fig. 3 shows the third catheter 5 with its spacer element 27 in the radially extended position. Fig. 4 shows a corresponding cross-sectional view.

The spacer element 57 comprises several, for example four wing elements 572, 574, 576, 578, as exemplarily depicted in Fig. 3, which may be formed by slots in the third catheter's wall extending in the first direction d1.

The third catheter 5 is fixed to the first catheter end portion 22 in an area 52 between a first end 59 of the longitudinal slots that faces a free end 53 of the third catheter 5 and the free end 53 of the third catheter 5. The section of the third catheter 5 away from the area 52 is a movable section of the third catheter 5. The spacer element 57 can be moved from its radially retracted position into its radially extended position by moving the movable section of the third catheter 5 relative to the second catheter 4 along the first direction d1.

As shown for example in Fig. 1, the third catheter 5 comprises an opening 54 which is co-aligned with the opening 26 of the first catheter end portion 22 to allow the second catheter end portion 42 to pass therethrough.

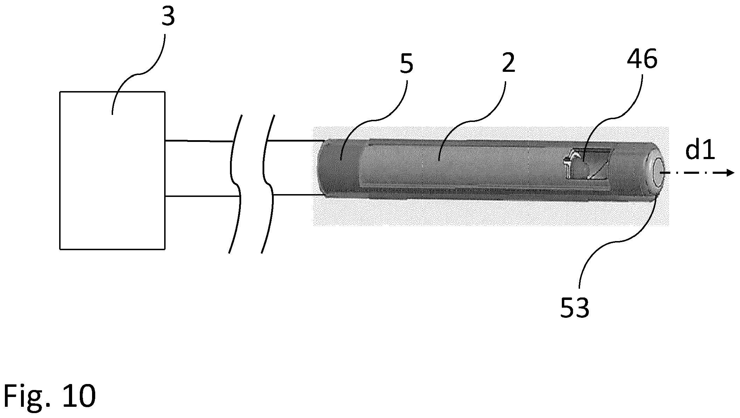

As sketched in Fig. 10, the catheter assembly further comprises a manipulation handle 3 that is coupled to the first catheter and that is configured to be disposed extracorporeal with respect to the patient.

Preferably, the manipulation handle comprises means for moving the second catheter 4 relative to the first catheter 2 and means for locking a position of the second catheter 4 relative to the first catheter 2. This facilitates handling during operation, since the second catheter end portion 42 may be fixed relative to the first catheter end portion 22, for example when it is in the second state or in the intermediate position.

Analogously, the manipulation handle 3 may comprise means for moving the cutting wire 6 relative to the first catheter 2 and means for locking a position of the cutting wire 6 relative to the first catheter 2. This may also help to fix the relative arrangement between the second catheter end portion 42 and the first catheter end portion 22.

Analogously, the manipulation handle 3 may comprise means for moving the puncture tip wire 48 relative to the second catheter 4 and means for locking a position of the puncture tip wire 48 relative to the second catheter 4.

Analogously, the manipulation handle 3 may comprise means for moving the third catheter 5 relative to the first catheter 2 and means for locking a position of the third catheter 5 relative to the first catheter 2.

A description of a possible process for using the catheter assembly is given next.

In an initial state of the catheter assembly, which may be also denominated as "hook-based dissection membrane cutting device", the third catheter spacer element 57 or wing elements 572, 574 of the third catheter 5 or "spacer catheter" as well as the second catheter spacer element 27 - hereinafter also referred to as "anchor element" 27 of the second catheter 4 or "working catheter" and the biased hook shape of the working catheter 4 itself are retracted within the first or "outer catheter" or "spacer catheter", so that the end portion of the catheter assembly forms at least essentially a cylindrical shape, as shown in Fig. 1 that can be inserted into a true lumen of an aorta descendens via a femoral access sheath. The outer diameter of the end portion in this state may be for example 10 to 12 Charriere, which corresponds to 3.33 to 4 mm.

While in this state, the catheter assembly is advanced to an upper point of the aorta where the dissection membrane has its upper limit in relation to the blood flow and slitting is to start. Contrast agent may be injected via the sheath catheter 2 to make the vessel more visible on the X-ray images during the procedure. X-ray markers at key points on various components of the catheter assembly may be used to make it possible to see the end portion and determine its orientation within the X-ray images.

Further, the assembly may be rotated axially, i. e. around the first direction d1, by turning the extracorporeal manipulation handle 3 of the assembly so that the opening 26 of the sheath catheter 2 faces the dissection membrane. Subsequently, the spacer wing elements 572, 574, 576, 578 may be unfolded - as illustrated in Fig. 3 - by relative coaxial translation between the spacer catheter 5 and the remaining components of the assembly, for example the sheath catheter 2 via the manipulation handle 3. The relative position may be fixed by a locking mechanism in the manipulation handle 3.

By gently pushing the assembly forwards and backwards, the spacer wing elements 572, 574, 576, 578 are aligned in the true lumen so that the opening 26 of the sheath catheter 2 is locked radially at a point where it faces the dissection membrane.

Both the puncture tip wire 48 and the cutting wire 6 may have a diameter of 0.3 mm and. With the exception of the puncture tip 46, and the cutting area of the cutting wire 6, these two wires 48, 6 are preferably electrically insulated over their entire length with a highly flexible and temperature-stable coating.

By advancing the working catheter 4 together with the puncture tip wire 46 and the cutting wire 6 using the manipulation handle 3 in relative translation to the rest of the assembly, the working catheter 4 moves out of the opening 26 of the sheath catheter 2. The puncture tip 46 is now applied to a high-frequency voltage of a monopolar electrocautery and thus serves as a cautery electrode. A standard adhesive electrode for electrocautery can be used as a neutral electrode.

From its tip starting, the working catheter 4 or its end portion 42 resumes to its actual hook shape and therefore presses against the membrane with its puncture tip 46.

As the working catheter 4 is advanced further, the puncture tip 46 punctures the dissection membrane and enters the false lumen of the aorta, continuously regaining its original hook shape.

As soon as the dissection membrane has been punctured, the voltage applied to the puncture wire can be switched off to prevent unintentional cauterization.

As soon as the working catheter 4 has completely regained its hook shape, the relative position of the working catheter 4 in the manipulation handle 3 can be fixed by a locking mechanism and the cutting wire 6 can be tightened via the manipulation handle 3, thereby stabilising the hook shape of the working catheter 4. The relative position of the cutting wire 6 can now also be fixed in the manipulation handle 3 using a locking mechanism. The cutting wire 6 thus forms a straight line between the edge of the sheath catheter 2 and the fixation point 64 on the working catheter 4. The inclination of the cutting wire 6, as illustrated exemplarily in Fig. 5, is selected so that the dissection membrane is pressed against the sheath catheter 2 during the slitting process.

For a further anchoring effect, the puncture wire 48 can now be tightened via the manipulation handle 3 to unfold the anchor wings 272, 274 of the working catheter 4, whereby the relative position of the puncture wire 48 in the manipulation handle 3 can be fixed by a locking mechanism. This improves the fixation of the hook and the cutting wire 6 to the dissection membrane and prevents the hook from slipping back into the actual, i. e. true lumen.

Contrast medium may be administered into the false lumen via the working catheter 4, while contrast medium may be administered into the true lumen via the sheath catheter 2.

The high-frequency voltage of the monopolar electrocautery can be applied to the cutting wire 6 so that the cutting wire 6 acts as a cautery electrode. The slitting procedure can now be performed by pulling the entire catheter assembly or its end portion towards the femoral access.

The spacer wings 572, 574, 576, 578 and the anchor wings 272, 274, as well as the shape of the working catheter 4 itself, minimise the risk of accidental cauterisation of aortic tissue on the sides of the dissection membrane. In addition, the pulling of the assembly or its end portion itself has a self-centering effect.

Once the slitting process is complete and the intended final position of the slit has been reached, the voltage applied to the cutting wire 6 may be switched off.

To fold up the assembly, the locks for the positions of the cutting wire 6, the puncture wire 48 and the working catheter 4 in the manipulation handle 3 must be released. By retracting the working catheter 4 in relative coaxial translation to the other device components via the manipulation handle 3, the cutting wire 6 slides back into the sheath catheter 2 and the anchor wings 272, 274 fold back into their original shape as soon as they reach the opening hole 26 of the sheath catheter 2.

After releasing the spacer catheter position lock on the manipulation handle 3, the spacer wings 572, 574, 576, 578 can be folded back into their original shape by relative coaxial translation between the spacer catheter 5 and the other components of the device.

The assembly has now regained its almost cylindrical shape, as illustrated in Fig. 1 and can be withdrawn from the femoral access sheath.

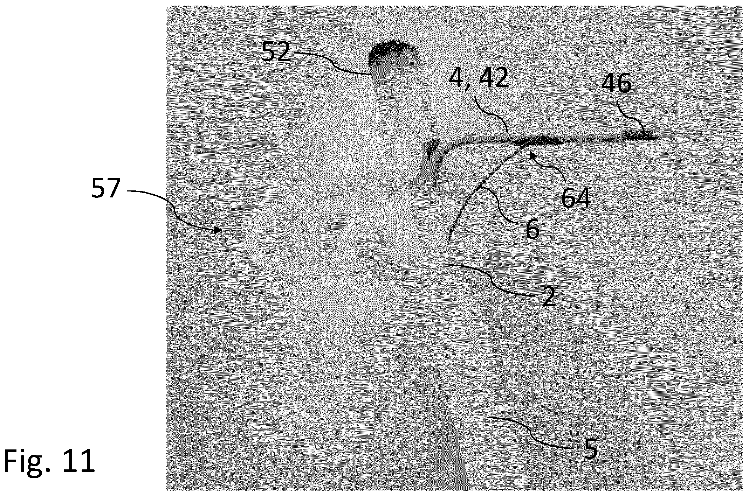

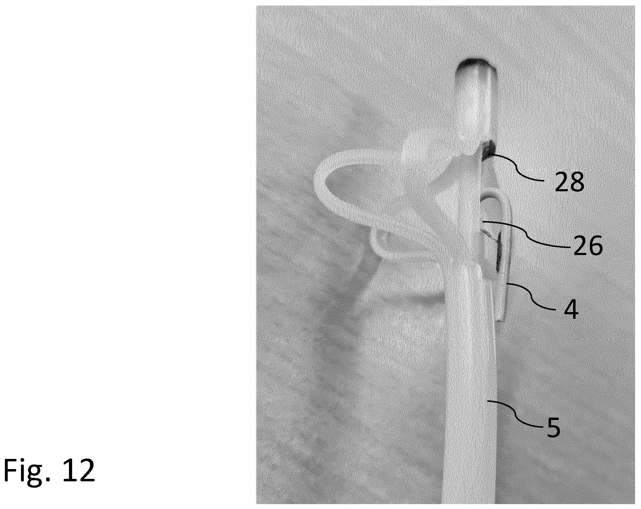

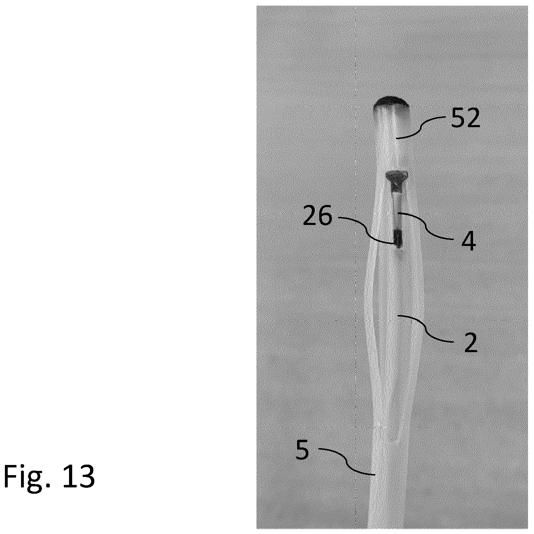

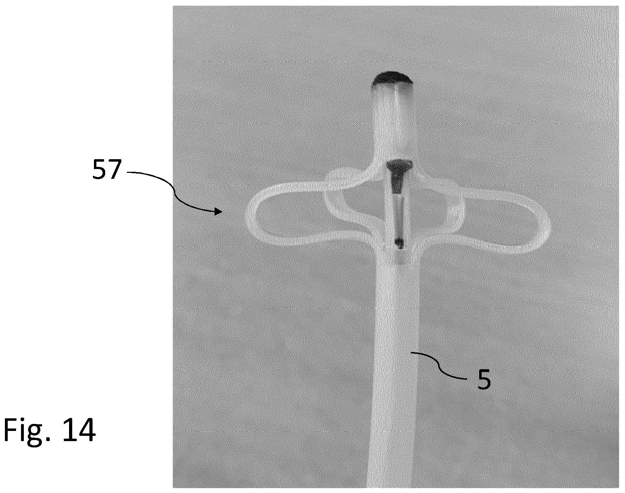

Figures 11 to 14 show another example of a catheter assembly according to the present description. The reference signs are used analogously here. Unless otherwise stated, the above explanations also apply with reference to this example.

One difference to the example above is that the second catheter end section 42 has no anchor element.

Another difference is that the first catheter 2 has non-circular cross-section. The cross-section may be at least essentially rectangular. The opening 26 may be formed preferably on the shorter side of the rectangular shape.

## Claims

1. A catheter assembly for slitting a dissection membrane of an aorta of a patient, comprising
an elongate first catheter (2), having a lumen (24) extending therethrough and having a first catheter end portion (22) configured to be inserted into a lumen of an aorta of a patient, the first catheter end portion (22) defining a first direction (d1) and comprising an opening (26), wherein the lumen (24) opens into an external space (A) via the opening (26),
an elongate second catheter (4) disposed movably within the lumen (24) of the first catheter (2) and having a second catheter end portion (42), the second catheter end portion (42) being movable relative to the first catheter (2) between a first state and a second state, wherein, when in the first state, the second catheter end portion (42) is disposed at least essentially within the lumen (24) of the first catheter (2), and, when in the second state, the second catheter end portion (42) extends through the opening (26) into the external space (A) outside of the first catheter (2),
wherein the second catheter end portion (42) is flexible and has a free end (44) defining a free end direction (df), and
wherein the second catheter end portion (42) is configured such that, when the second catheter end portion (42) is in the first state, the free end (44) assumes a first orientation relative to the first catheter end portion (22), and, when the second catheter end portion (42) is in the second state, the free end (44) assumes a second orientation relative to the first catheter end portion (22), wherein the second orientation differs from the first orientation.

2. The catheter assembly of claim 1,
wherein the second catheter end portion (42) is biased such that the free end (44) assumes the second orientation relative to the first catheter end portion (22) when the second catheter end portion (42) is in the second state.

3. The catheter assembly of any of the preceding claims,
wherein, when the second catheter end portion (42) is in the second state, the free end direction (d1) is at least essentially opposite to the first direction (d1).

4. The catheter assembly of any of the preceding claims,
wherein, when the second catheter end portion (42) is in the first state, the free end direction (df) is at least essentially co-aligned with the first ditrection (d1).

5. The catheter assembly of any of the preceding aspects,
wherein the first catheter end portion (22) comprises a tubular wall, and wherein the opening (26) is an opening formed in the tubular wall, and/or
wherein the first catheter end portion (22) comprises a guiding structure (28) configured to laterally deflect or assist in laterally deflecting the free end (44) of the second catheter end portion (42) with respect to the first direction (d1), when the second catheter end portion (42) is moved from the first state into the second state.

6. The catheter assembly of any of the preceding aspects, further comprising
a cutting wire (6) extending through the lumen (24) of the first catheter (2), the cutting wire (6) being fixed to a fixation point (64) on the second catheter end portion (42) and being configured to be energized with electrical current.

7. The catheter assembly of claim 6,
wherein, when the free end (44) is in the second orientation, the cutting wire (6) extends through the opening (26) of the first catheter end portion (22) into the external space (A) and to the fixation point (64) on the second catheter end portion (42).

8. The catheter assembly of any of the preceding claims,
wherein the free end (44) of the second catheter end portion (42) comprises a puncture tip (46) configured to puncture the dissection membrane of the aorta,
preferably wherein the puncture tip (46) is configured to be energized with electrical current such that the dissection membrane can be punctured by the puncture tip (46) via electrical current.

9. The catheter assembly of claim 8, further comprising
a puncture tip wire (48) for supplying electrical energy to the puncture tip (46).

10. The catheter assembly of claim 9,
wherein the second catheter (4) has a lumen (41) extending therethrough, wherein the puncture tip wire (48) is disposed movably within the lumen (41) of the second catheter (4).

11. The catheter assembly of any of the preceding claims,
wherein the second catheter (2) comprises an spacer element (27) configured to be moved radially inwards and outwards with respect the free end direction (df) between a first, radially retracted position and a second, radially extended position,
preferably wherein the anchor element (27) comprises two wing elements (272, 274) which are arranged on two opposite sides with respect to the free end direction (df).

12. The catheter assembly of claim 11, comprising the features of claim 9,
wherein the anchor element (27) can be moved from its first, radially retracted position into its second, radially extended position by manipulating the puncture tip wire (48).

13. The catheter assembly of any of the preceding claims,
further comprising an elongate third catheter (5),
the third catheter (5) comprising a spacer element (57) configured to be moved radially inwards and outwards with respect to the first direction (d1) between a first, radially retracted position and a second, radially extended position.

14. The catheter assembly of claim 13,
wherein the spacer element (57) comprises at least two wing elements (572, 574, 576, 578) formed by longitudinal slots within the third catheter (5), preferably by slots extending in the first direction (d1).

15. The catheter assembly of any of the preceding claims, further comprising
a manipulation handle (3), coupled to the first catheter (2) and configured to be disposed extracorporeal with respect to the patient,
wherein the manipulation handle (3) comprises means for locking a position of the second catheter (4) relative to the first catheter (2), and/or
wherein the manipulation handle (3) comprises means for locking a position of the cutting wire (6) relative to the first catheter (2), and/or
wherein the manipulation handle (3) comprises means for locking a position of the puncture tip wire (48) relative to the second catheter (4), and/or
wherein the manipulation handle (3) comprises means for locking a position of the third catheter (5) relative to the first catheter (2).
